# FASCICULE DE BREVET EUROPEEN

(11) **EP 4 056 515 B1**
(45) Date de publication et mention de la délivrance du brevet: **03.12.2025**
(21) Numéro de dépôt: 22160127.1
(22) Date de dépôt: 04.03.2022
(51) Int. Cl.: B66C 11/18, B66C 15/00, B66C 13/46, B66C 23/26, B66D 3/04, E21C 29/14, D07B 1/14, G01N 33/20

(54) **PROCÉDÉ D ASSISTANCE DE MAINTENANCE D'UN CÂBLE MÉTALLIQUE D'UN APPAREIL DE LEVAGE OU DE TRANSPORT**
VERFAHREN ZUR UNTERSTÜTZUNG DER WARTUNG EINES DRAHTSEILS EINER HEBE- ODER TRANSPORTVORRICHTUNG
METHOD FOR ASSISTANCE IN MAINTAINING A METAL CABLE OF A DEVICE FOR LIFTING OR TRANSPORT

(30) Priorité: 09.03.2021 FR 2102286
(43) Date de publication de la demande: 14.09.2022
(73) Titulaire: Manitowoc Crane Group France, 69570 Dardilly (FR)
(72) Inventeur: RONI-DAMOND, Bruno, 69002 LYON (FR); CHALAVON, Jonathan, 69780 MIONS (FR); BLASIAK, Jérémy, 69210 FLEURIEUX SUR L'ARBRESLE (FR); BUCAS, Simon, 69002 Lyon (FR); BEYLIER, Sébastien, 69760 LIMONEST (FR)
(74) Mandataire: Germain Maureau

(56) Documents cités:
- EP-A2- 2 740 704
- WO-A1-2015/139842
- JP-A- 2005 134 261
- JP-A- H07 112 894
- KR-A- 20160 065 699

## Description

L'invention se rapporte à un procédé d'assistance de maintenance pour assister une maintenance d'un câble métallique d'un appareil de levage ou de transport, et en particulier un câble métallique qui forme une boucle fermée et qui coopère avec un système de remise en tension.

Un câble métallique en boucle fermée est un câble qui présente un cheminement en boucle fermée et qui a une longueur constante en utilisation, aux allongements près qui interviennent au fur et à mesure des sollicitations sur le câble métallique. Un tel câble métallique en boucle fermée présente généralement deux extrémités fixées sur un même tambour d'enroulement d'un treuil, pour assurer un déplacement d'un élément fonctionnel de l'appareil de levage ou de transport.

L'invention trouve une application favorite, et non limitative, pour un appareil de levage de type grue, et notamment une grue à tour, une grue à montage par éléments, une grue à montage automatisé, une grue portuaire et une grue mobile. L'invention peut aussi s'appliquer à un appareil de transport à câble, fonctionnant avec un câble métallique en boucle fermée, comme par exemple un téléphérique ou un télésiège.

Dans une application à un appareil de levage, tel qu'une grue, il est connu d'employer un câble métallique en boucle fermée pour déplacer en translation un chariot distributeur le long d'une flèche, un tel câble étant appelé câble de distribution, et où une charge à lever est suspendue sur un tel chariot distributeur par un câble de levage pour la faire monter et descendre vis-à-vis du chariot distributeur, permettant ainsi une distribution de la charge (déplacement le long de la flèche) et un levage de la charge (montée et descente de la flèche). Il est à noter qu'un tel câble de levage ne forme pas une boucle fermée, mais forme une boucle ouverte en ayant qu'une extrémité fixée sur le tambour d'enroulement d'un treuil de levage.

De manière générale, les câbles métalliques sont soumis à un endommagement par fatigue. Le déposant a observé que ce phénomène de fatigue est influencé par le niveau de tension dans le câble métallique, généralement induit par la charge, et aussi par les opérations de remise en tension du câble métallique qui interviennent dans la durée de vie du câble métallique.

En effet, un câble métallique en boucle fermée présente généralement une grande longueur, pouvant dépasser les cent mètres selon par exemple les longueurs de flèche, qui contribue à ce que le câble métallique s'allonge pendant le temps de son utilisation, de sorte qu'il est indispensable de retendre ce câble métallique régulièrement. Généralement, il est nécessaire d'effectuer cette remise en tension du câble métallique environ deux à trois fois pendant le premier mois d'utilisation de la grue, puis ensuite une fois par trimestre environ. Classiquement, un système de remise en tension, aussi appelé mécanisme tendeur, est couplé à un tel câble métallique en boucle fermée pour assurer ce réglage de la tension du câble métallique, où ce système de remise en tension peut par exemple et à titre non limitatif comprendre un dispositif à tambour différentiel.

Cette remise en tension du câble métallique en boucle fermée est en effet nécessaire pour assurer un bon fonctionnement du déplacement de l'élément fonctionnel associé, comme par exemple la translation du chariot distributeur ou l'inclinaison de la flèche relevable, qui n'accepte pas d'avoir un câble métallique détendu. Il est d'ailleurs parfois prévu un système de sécurité du mou de câble, en particulier pour les câbles de distribution, qui entre en action et bloque le chariot distributeur en position de sécurité conformément aux exigences classiques de sécurité, et en particulier aux indications de la norme européenne EN 2 14439 sur « appareils de levage a charge suspendue - sécurité - grues à tour ».

Ces opérations de remise en tension du câble métallique sont également nécessaires pour garantir un fonctionnement réactif du déplacement de l'élément fonctionnel associé et un positionnement précis de cet élément fonctionnel. En effet, à chaque impulsion de déplacement, l'élément fonctionnel réagit plus vite lorsque le câble métallique est suffisamment tendu car il n'y aura pas ou peu de mou de câble à récupérer.

L'état de la technique peut également être illustré par les enseignements du document JP 2005 134261 qui propose un procédé d'assistance de maintenance pour assister une maintenance d'un câble métallique de levage (donc en boucle ouverte) d'une grue, mettant en œuvre un test de traction sur un câble étalon du même type afin de recueillir des données de taux d'allongement et des données de résistance résiduelle, pour déterminer si le câble métallique utilisé a atteint la fin de sa durée de vie.

Il est aussi connu du document EP 2 740 704 de mettre en œuvre une détection d'usure d'un câble de levage (donc en boucle ouverte) en fibre haute résistance d'une grue en fonction d'un allongement du câble, pour produire un signal de dépose en cas de dépassement d'un allongement maximal admissible.

Dans le cas des câbles métalliques, les normes définissent les limites d'endommagement ou d'usure acceptables pour chaque câble, ces limites étant souvent données en nombre de fils coupés observés sur une longueur prédéfinie du câble. Aussi, pour des raisons évidentes de sécurité, des opérations de maintenance interviennent régulièrement pour inspecter visuellement les câbles dans un appareil de levage ou de transport, et en particulier les câbles métalliques, avec cependant de nombreuses difficultés : le câble métallique peut être particulièrement long et présenter des sections parfois difficiles d'accès, certains types de câble dont les câbles anti-giratoires peuvent être endommagés avec des coupures de fils intérieurs et donc non visibles lors d'une inspection.

Ainsi, bien que la plupart du temps ce type d'inspection visuelle soit efficace, il existe un réel besoin de fiabiliser et d'automatiser la maintenance préventive des câbles métalliques en boucle fermée pour estimer un indice d'endommagement et ainsi procéder au remplacement des câbles trop fragilisés lorsque nécessaire.

A cette fin, l'invention propose un procédé d'assistance de maintenance pour assister une maintenance d'un câble métallique d'un appareil de levage ou de transport, ledit câble métallique étant un câble métallique formant une boucle fermée et coopérant avec un système de remise en tension, ledit procédé d'assistance de maintenance mettant en œuvre les étapes suivantes :
- surveillance des opérations de remise en tension du câble métallique par le système de remise en tension, qui interviennent dans la durée de vie du câble métallique ;
- pour chaque opération de remise en tension du câble métallique, détermination d'une valeur d'allongement du câble métallique ;
- estimation d'une valeur d'allongement global du câble métallique, qui correspond à la somme des valeurs d'allongement du câble métallique déterminées après chaque opération de remise en tension ;
- détermination d'un indice d'endommagement du câble métallique en fonction de la valeur d'allongement global.

Ainsi, l'invention se base sur une estimation d'une valeur d'allongement global du câble métallique pour déterminer l'indice d'endommagement, et le cas échéant alerter un opérateur ; étant noté que l'invention s'appuie sur des valeurs d'allongement après chaque opération de remise en tension, ce qui est particulièrement avantageux car ces opérations de remise en tension sont particulièrement adaptées pour pouvoir apprécier ces valeurs d'allongement, dans la mesure où une remise en tension vise justement à rattraper un mou de câble qui résulte justement d'un allongement du câble.

Il est à noter que, au sens de l'invention, le câble métallique formant une boucle fermée peut être composé d'un seul câble métallique en boucle fermée ou de deux câbles métalliques qui forment ensemble une boucle fermée.

Selon une caractéristique, la détermination de l'indice d'endommagement du câble métallique est aussi fonction du nombre d'opérations de remise en tension du câble métallique.

En effet, le nombre de remise en tension joue sur l'endommagement du câble métallique, et sa prise en considération est particulièrement avantageuse dans le cadre de cette surveillance préventive.

Selon une autre caractéristique, la détermination de l'indice d'endommagement du câble métallique est aussi fonction des intervalles de temps entre les opérations successives de remise en tension du câble métallique.

En effet, plusieurs opérations de remise en tension dans un intervalle de temps réduit, par exemple à quelques jours d'intervalle, montreraient une anomalie, et donc un endommagement potentiel qui sera pris en considération dans l'établissement de l'indice d'endommagement.

Selon une possibilité, le procédé comprend, après chaque opération de remise en tension du câble métallique, une comparaison de l'indice d'endommagement du câble métallique avec un seuil d'endommagement prédéfini, et une émission d'une alerte si l'indice d'endommagement du câble métallique dépasse ledit seuil d'endommagement.

Selon une autre possibilité, l'indice d'endommagement correspond à la valeur d'allongement global, et le seuil d'endommagement correspond à un seuil maximal d'allongement.

Autrement dit, c'est la valeur d'allongement global qui servira pour l'indice d'endommagement.

Selon une caractéristique, le câble métallique présente deux extrémités fixées sur un même tambour d'enroulement d'un treuil, pour assurer un déplacement d'un élément fonctionnel de l'appareil de levage ou de transport.

Un tel câble métallique peut par exemple être composé de deux brins qui s'enroulent sur un même tambour d'enroulement et qui sont fixés sur le même élément fonctionnel. Ainsi, chaque brin présente une première extrémité fixée sur le tambour d'enroulement, et une seconde extrémité fixée sur l'élément fonctionnel. Les premières extrémités des deux brins forment ainsi les extrémités du câble métallique, et ces deux brins forment bien une boucle fermée.

Dans une réalisation particulière, le câble métallique est un câble métallique de distribution assurant un déplacement d'un chariot distributeur déplaçable le long d'une flèche d'un appareil de levage ou de transport.

Un tel câble métallique de distribution est généralement composé de deux brins qui s'enroulent sur un même tambour d'enroulement et qui sont fixés sur le même chariot distributeur pour former une boucle fermée.

Avantageusement, la surveillance des opérations de remise en tension du câble métallique de distribution met en œuvre :
- une surveillance d'un treuil de distribution comprenant un tambour d'enroulement sur lequel s'enroulent un brin arrière et un brin avant du câble métallique de distribution pour déplacer le chariot distributeur, ladite surveillance du treuil de distribution comprenant une détermination d'une position théorique du chariot distributeur en fonction d'une mesure d'enroulement du brin arrière ou du brin avant sur le tambour d'enroulement ; et
- une surveillance d'une position réelle du chariot distributeur sur la flèche ;
et dans lequel, pour chaque opération de remise en tension du câble métallique de distribution, la détermination d'une valeur d'allongement du câble métallique de distribution consiste à établir la valeur d'allongement du câble métallique de distribution comme correspondant à la différence entre la position théorique du chariot distributeur et la position réelle du chariot distributeur.

Ainsi, les valeurs d'allongement seront déterminées en comparant, lors de chaque opération de remise en tension, la position théorique du chariot distributeur et la position réelle du chariot distributeur. La position théorique du chariot distributeur correspond à la position que devrait avoir le chariot distributeur si le câble métallique de distribution ne s'était pas allongé depuis la précédente remise en tension, et cette position théorique est établie en se basant sur une mesure d'enroulement du brin arrière et/ou du brin avant sur le tambour. La position réelle du chariot distributeur correspond à la vraie position du chariot distributeur, qui peut être mesurée au moyen d'un capteur placé sur la flèche et/ou sur le chariot distributeur.

Avantageusement, après chaque opération de remise en tension du câble métallique de distribution, est mis en œuvre une mise à zéro de la position théorique du chariot distributeur afin de calibrer cette position théorique du chariot distributeur sur la position réelle du chariot distributeur.

Ainsi cette mise à zéro permet de recaler automatiquement le « zéro portée » du chariot distributeur qui correspond à la position de référence du chariot distributeur pour déterminer sa position le long de la flèche. Avec l'automatisation de cette mise à zéro, le risque d'erreur sur cette position de référence est supprimé et l'indication de portée restera toujours juste et fiable.

Selon une possibilité, l'alerte se présente sous la forme d'un signal d'alerte visuel ou sonore sur une interface de pilotage, comme par exemple une interface de pilotage pour le grutier ou pour un service de surveillance distant.

L'invention se rapporte également à un appareil de levage ou de transport comprenant un câble métallique qui forme une boucle fermée et qui coopère avec un système de remise en tension, ladite grue comprenant :
- un système de surveillance pour surveiller des opérations de remise en tension du câble métallique par le système de remise en tension, qui interviennent dans la durée de vie du câble métallique ;
- un système de contrôle/commande configuré pour :
- déterminer une valeur d'allongement du câble métallique après chaque opération de remise en tension du câble métallique ;
- estimer une valeur d'allongement global du câble métallique, qui correspond à la somme des valeurs d'allongement du câble métallique déterminées après chaque opération de remise en tension ;
- déterminer un indice d'endommagement du câble métallique en fonction de la valeur d'allongement global.

Dans un mode de réalisation particulier, l'appareil de levage ou de transport comprend un treuil muni d'un tambour d'enroulement et le câble métallique présente deux extrémités fixées sur ce tambour d'enroulement, pour assurer un déplacement d'un élément fonctionnel de l'appareil de levage ou de transport.

Dans une réalisation particulière, le câble métallique est un câble métallique de distribution assurant un déplacement d'un chariot distributeur déplaçable le long d'une flèche de l'appareil de levage ou de transport.

Selon une possibilité, le système de contrôle/commande est configuré pour déterminer l'indice d'endommagement du câble métallique aussi en fonction du nombre d'opérations de remise en tension du câble métallique.

Selon une autre possibilité, le système de contrôle/commande est configuré pour déterminer l'indice d'endommagement du câble métallique aussi en fonction des intervalles de temps entre les opérations successives de remise en tension du câble métallique.

Dans une réalisation avantageuse, l'appareil de levage ou de transport comprend un système d'alerte relié au système de contrôle/commande, ledit système de contrôle/commande étant configuré pour, après chaque opération de remise en tension du câble métallique, comparer l'indice d'endommagement du câble métallique avec un seuil d'endommagement prédéfini, et pour activer le système d'alerte afin d'émettre une alerte si l'indice d'endommagement du câble métallique dépasse ledit seuil d'endommagement.

D'autres caractéristiques et avantages de la présente invention apparaîtront à la lecture de la description détaillée ci-après, d'un exemple de mise en œuvre non limitatif, faite en référence aux figures annexées dans lesquelles :
La Figure 1 est une vue schématique d'une grue selon l'invention ;
La Figure 2 est une vue schématique d'une séquence d'étapes mise en œuvre dans le procédé d'assistance de maintenance selon l'invention.

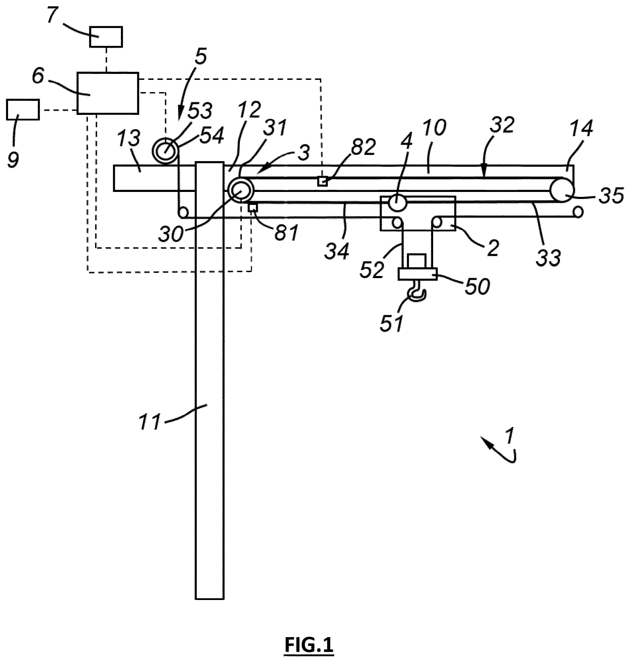

En référence à la Figure 1, une grue 1 selon l'invention, par exemple de type grue à tour, comprend une flèche 10 distributrice montée sur une tour 11 (aussi appelée mât) au pied 12 de la flèche 10. De manière classique, le pied 12 de la flèche 10 est montée rotative sur la tour 11 selon un axe vertical. La flèche 10 peut être prolongée de l'autre côté de la tour 11 par une contre-flèche 13, généralement muni de lests. Ainsi, la flèche 10 et la contre-flèche 13 forment une partie tournante fixée à un pivot, aussi appelé orient, faisant la liaison entre cette partie tournante et la tour 11 ; un tel pivot intégrant un système motorisé d'orientation pour actionner la rotation de la partie tournante selon l'axe vertical.

La grue 1 comporte en outre un chariot distributeur 2 conformé pour distribuer une charge (non illustrée) le long de la flèche 10, ce chariot distributeur 2 circulant sur un chemin de roulement formé sur la flèche 10, entre le pied 12 et la pointe 14 de la flèche 10, aussi appelée extrémité libre de la flèche 10.

Ce chariot distributeur 2 est reliée à un treuil de distribution 3 propre à déplacer le chariot distributeur 2 le long du chemin de roulement selon une direction avant (autrement dit en direction de la pointe 14 de la flèche 10, vers la droite sur la figure) et une direction arrière (autrement dit en direction du pied 12 de la flèche 10, vers la gauche sur la figure) opposées. Ce treuil de distribution 3 est disposé de préférence sur la flèche 10.

Comme schématisé, ce treuil de distribution 3 comprend un moteur de distribution 30 entrainant un tambour d'enroulement 31 accouplé à un câble métallique de distribution 32 ayant ces deux extrémités fixées sur ce tambour d'enroulement 31. Ce câble métallique de distribution 32 présente ainsi un brin avant 33 (aussi appelé câble avant) et un brin arrière 34 (aussi appelé câble arrière) fixés de part et d'autre du chariot distributeur 2. Le brin avant 33 et le brin arrière 34 s'enroulent tous les deux sur le tambour d'enroulement 31, et le brin avant 33 circule depuis le tambour d'enroulement 31 jusqu'à une poulie avant 35 disposée sur la pointe 14 de la flèche 10 avant de revenir jusqu'à l'avant du chariot distributeur 2, tandis que le brin arrière 34 circule depuis le tambour d'enroulement 31 jusqu'à l'arrière du chariot distributeur 2. Ainsi, le câble métallique de distribution 32 est formé du brin avant 33 et du brin arrière 34 qui sont deux câbles métalliques enroulés sur le même tambour d'enroulement 31 et fixés sur le même chariot distributeur 2 pour, ensemble, former une boucle fermée.

Le brin avant 33 et/ou le brin arrière 34 du câble métallique de distribution 32 est ou sont accouplés à un système de remise en tension 4 porté par le chariot distributeur 2. Ce système de remise en tension 4 forme un mécanisme tendeur, à actionnement manuel ou automatisé, qui est couplé au câble métallique de distribution 32 pour assurer un réglage de la tension du câble métallique de distribution 32. A titre d'exemple non limitatif, ce système de remise en tension 4 est un dispositif à tambour différentiel. Ce dispositif à tambour différentiel peut par exemple être utilisé pour « raccourcir » le brin avant 33 uniquement (ou le brin arrière 34 uniquement) tout en contribuant à ce que les deux brins, avant 33 et arrière 34, soient retendus en même temps car ils forment à eux deux une boucle fermée. Ce câble métallique de distribution 32 forme ainsi une boucle fermée, sa longueur restant constante pendant les déplacements du chariot distributeur 2, aux allongements près qui interviennent au fur et à mesure des sollicitations sur le câble métallique de distribution 32.

Ce chariot distributeur 2 supporte également des poulies qui assurent le guidage d'un câble de levage 52 qui supporte un dispositif de moufle 50, qui est ainsi suspendu au chariot distributeur 2 par le câble de levage 52. Ce dispositif de moufle 50 supporte un organe de levage 51 prévu pour un accrochage de la charge et pouvant se présenter sous la forme d'un crochet articulé sur le dispositif de moufle 50. La grue 1 comprend un treuil de levage 5 muni d'un moteur de levage 53 entrainant un tambour de levage 54 sur lequel est fixée une seule extrémité du câble de levage 52. Ce câble de levage 52 passe donc sur les poulies du chariot distributeur 2 et circule jusqu'à la pointe 14 de la flèche 10. Ce câble de levage 52 forme ainsi une boucle ouverte, sa longueur étant variable par enroulement/déroulement autour du tambour de levage 54 pour permettre de lever/descendre la charge.

La grue 1 comprend un système de contrôle/commande 6 qui est relié au treuil de distribution 3 et au treuil de levage 5 pour un contrôle de la vitesse moteur et du sens moteur du moteur de distribution 30, en distribution avant et arrière, et également du moteur de levage 53, en montée et en descente, et ainsi pour un contrôle du déplacement de la charge. Ce système de contrôle/commande 6 est également relié à un dispositif de pilotage 7, servant au pilotage des fonctions de la grue 1, par un pilote ou grutier, de sorte que le système de contrôle/commande 6 reçoit des consignes en provenance du dispositif de pilotage 7 pour piloter les moteurs 30, 53.

Ce système de contrôle/commande 6 est relié à un système de surveillance pour surveiller des opérations de remise en tension du câble métallique de distribution 32 par le système de remise en tension 4, qui interviennent dans la durée de vie du câble métallique de distribution 32.

Ce système de surveillance comprend un premier capteur 81 positionné au niveau du tambour d'enroulement 31 du treuil de distribution 3 pour détecter une mesure d'enroulement du brin arrière 34 ou du brin avant 33 sur le tambour d'enroulement 31. Ce premier capteur 81 permet ainsi de réaliser une surveillance du treuil de distribution 3 par la mise en œuvre d'une détermination d'une position théorique XTH du chariot distributeur 2 en fonction d'une telle mesure d'enroulement du brin arrière 34 ou du brin avant 33 sur le tambour d'enroulement 31.

Ce système de surveillance comprend un second capteur 82 positionné sur la flèche 10 le long du câble métallique de distribution 32, par exemple au niveau du pied 12 de la flèche 10, pour détecter une position réelle XRL du chariot distributeur 2.

Après une remise en tension du câble métallique de distribution 32, le système de contrôle/commande 6 effectue une calibration ou une mise à zéro de la position théorique XTH du chariot distributeur 2 afin de calibrer cette position théorique XTH du chariot distributeur 2 sur la position réelle XRL du chariot distributeur, autrement dit XTH = XRL à l'issue de cette mise à zéro. Le système de contrôle/commande 6 suit ainsi chaque opération de remise en tension du câble métallique de distribution 32, par l'occurrence d'un réajustement de la position théorique XTH.

Ce système de contrôle/commande 6 est configuré pour :
- après chaque opération de remise en tension du câble métallique de distribution 32, déterminer une valeur d'allongement ΔL du câble métallique de distribution 32 correspondant à la différence entre la position théorique XTH du chariot distributeur et la position réelle XRL du chariot distributeur 2 avant l'opération remise en tension, autrement dit ΔL = XRL - XTH ;
- estimer une valeur d'allongement global ΔLG du câble métallique de distribution 32, qui correspond à la somme des valeurs d'allongement ΔL du câble métallique de distribution 32 déterminées après chaque opération de remise en tension ;
- déterminer un indice d'endommagement IND du câble métallique de distribution 32 en fonction de la valeur d'allongement global ΔLG.

Il est à noter que l'indice d'endommagement IND peut aussi être calculé en fonction du nombre d'opérations de remise en tension du câble métallique de distribution 32 et/ou des intervalles de temps entre les opérations successives de remise en tension du câble métallique de distribution 32 (autrement dit la fréquence des opérations de remise en tension). Par ailleurs, cet indice d'endommagement IND peut correspondre directement à la valeur d'allongement global ΔLG, soit IND=ΔLG, avec optionnellement des pondérations associées au nombre d'opérations de remise en tension et/ou aux intervalles de temps entre les opérations successives de remise en tension.

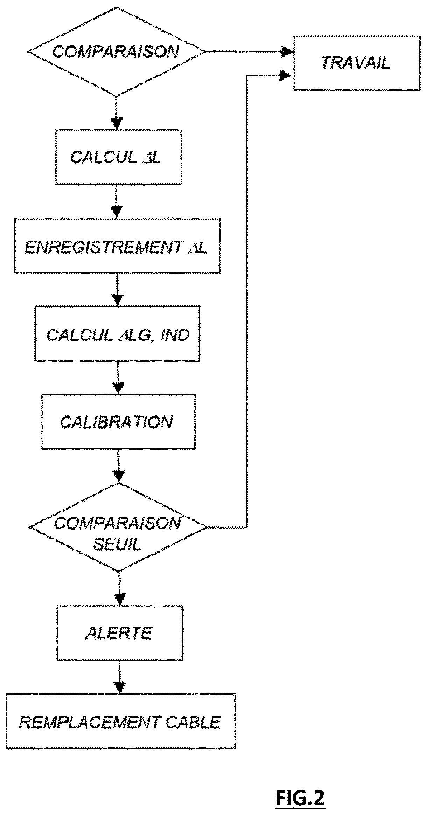

La Figure 2 illustre des étapes mises en œuvre pour cette grue 1 pour un procédé d'assistance de maintenance du câble métallique de distribution 32, qui commence par une étape « COMPARAISON » qui consiste à comparer la position théorique XTH et la position réelle XRL, si la position théorique XTH correspond à la position réelle XRL (soit XTH = XRL) alors les opérations de travail de levage/distribution de la charge sont possibles dans une phase « TRAVAIL », par contre si la position théorique XTH ne correspond pas à la position réelle XRL (soit XTH ≠ XRL) alors une phase de remise en tension et de détermination de l'indice d'endommagement IND est initiée.

La phase de remise en tension et de détermination de l'indice d'endommagement IND commence par une étape « CALCUL ΔL » qui consiste à calculer la valeur d'allongement ΔL qui est égal à la différence entre la position théorique XTH et la position réelle XRL, suivie d'une étape « ENREGISTREMENT ΔL » qui consiste à enregistrer la valeur d'allongement ΔL dans une mémoire du système de contrôle/commande 6. Il est à noter que ces valeurs d'allongement ΔL sont supprimées ou remises à zéro lors d'un remplacement du câble métallique de distribution 32.

Ensuite, une étape « CALCUL ΔLG, IND » consiste à calculer la valeur d'allongement global ΔLG qui correspond à la somme des valeurs d'allongement ΔL enregistrées dans la mémoire, et ensuite à calculer l'indice d'endommagement IND en fonction de cette valeur d'allongement global ΔLG. Puis, une étape « CALIBRATION » consiste à calibrer la position théorique XTH pour correspondre à la position réelle XRL (soit XTH = XRL). Puis une étape « COMPARAISON SEUIL » qui consiste à comparer l'indice d'endommagement IND avec un seuil d'endommagement LIM prédéfini. Si l'indice d'endommagement IND est inférieur au seuil d'endommagement LIM (ce qui traduit que l'indice d'endommagement IND est faible et le câble est toujours utilisable) alors les opérations de travail de levage/distribution de la charge sont possibles dans la phase « TRAVAIL », par contre si l'indice d'endommagement IND est supérieur au seuil d'endommagement LIM (ce qui traduit que l'indice d'endommagement IND est élevé et le câble est trop endommagé pour être utilisable) alors est mise en œuvre une étape « ALERTE » qui consiste à émettre une alerte pour avertir un opérateur, comme le grutier, de la situation pour ensuite déclencher une phase « REMPLACEMENT CABLE » consistant à déposer le câble métallique de distribution 32 pour le remplacer par un nouveau.

Ainsi, la grue 1 comprend un système d'alerte 9 relié au système de contrôle/commande 6 et configuré pour émettre une alerte lorsqu'activé. Ce système d'alerte 9 peut se présenter sous la forme d'un affichage visuel, par exemple au niveau d'une interface de pilotage placée dans une cabine de pilotage ou d'une interface distante, de sorte que le signal d'alerte est un signal visuel sur cette interface de pilotage. En variante ou en complément, ce système d'alerte 9 peut comprendre un émetteur sonore, par exemple dans la cabine de pilotage, de sorte que le signal d'alerte est un signal sonore.

L'invention décrite ci-dessus s'applique donc à un câble métallique de distribution 32 d'une grue, mais elle peut également s'appliquer à d'autres types de câble métallique en boucle fermée et/ou à d'autres types d'appareil de levage ou de transport fonctionnant par câble.

## Revendications

1. Procédé d'assistance de maintenance pour assister une maintenance d'un câble métallique (32) d'un appareil de levage ou de transport (1), ledit procédé d'assistance de maintenance étant **caractérisé en ce que** ledit câble métallique (32) est un câble métallique formant une boucle fermée et coopérant avec un système de remise en tension (4), et **en ce qu'**il met en œuvre les étapes suivantes :
- surveillance des opérations de remise en tension du câble métallique (32) par le système de remise en tension (4), qui interviennent dans la durée de vie du câble métallique (32) ;
- pour chaque opération de remise en tension du câble métallique (32), détermination d'une valeur d'allongement (ΔL) du câble métallique (32) ;
- estimation d'une valeur d'allongement global (ΔLG) du câble métallique (32), qui correspond à la somme des valeurs d'allongement (ΔL) du câble métallique (32) déterminées après chaque opération de remise en tension ;
- détermination d'un indice d'endommagement (IND) du câble métallique (32) en fonction de la valeur d'allongement global (ΔLG).

2. Procédé d'assistance de maintenance selon la revendication 1, dans lequel la détermination de l'indice d'endommagement (IND) du câble métallique (32) est aussi fonction du nombre d'opérations de remise en tension du câble métallique (32).

3. Procédé d'assistance de maintenance selon la revendication 1 ou 2, dans lequel la détermination de l'indice d'endommagement (IND) du câble métallique (32) est aussi fonction des intervalles de temps entre les opérations successives de remise en tension du câble métallique (32).

4. Procédé d'assistance de maintenance selon l'une quelconque des revendications 1 à 3, comprenant, après chaque opération de remise en tension du câble métallique (32), une comparaison de l'indice d'endommagement (IND) du câble métallique (32) avec un seuil d'endommagement (LIM) prédéfini, et une émission d'une alerte si l'indice d'endommagement (IND) du câble métallique (32) dépasse ledit seuil d'endommagement (LIM).

5. Procédé d'assistance de maintenance selon la revendication 4, dans lequel l'indice d'endommagement (IND) correspond à la valeur d'allongement global (ΔLG), et le seuil d'endommagement (LIM) correspond à un seuil maximal d'allongement.

6. Procédé d'assistance de maintenance selon la revendication 4 ou 5, dans lequel l'alerte se présente sous la forme d'un signal d'alerte visuel ou sonore sur une interface de pilotage.

7. Procédé d'assistance de maintenance selon l'une quelconque des revendications 1 à 6, dans lequel le câble métallique (32) présente deux extrémités fixées sur un même tambour d'enroulement (31) d'un treuil (3), pour assurer un déplacement d'un élément fonctionnel (2) de l'appareil de levage ou de transport (1).

8. Procédé d'assistance de maintenance selon l'une quelconque des revendications 1 à 7, dans lequel le câble métallique est un câble métallique de distribution (32) assurant un déplacement d'un chariot distributeur (2) déplaçable le long d'une flèche (10) de l'appareil de levage ou de transport (1).

9. Procédé d'assistance de maintenance selon la revendication 8, dans lequel la surveillance des opérations de remise en tension du câble métallique de distribution (32) met en œuvre :
- une surveillance d'un treuil de distribution (3) comprenant un tambour d'enroulement (31) sur lequel s'enroulent un brin arrière (34) et un brin avant (33) du câble métallique de distribution (32) pour déplacer le chariot distributeur (2), ladite surveillance du treuil de distribution (3) comprenant une détermination d'une position théorique (XTH) du chariot distributeur (2) en fonction d'une mesure d'enroulement du brin arrière (34) ou du brin avant (33) sur le tambour d'enroulement ; et
- une surveillance d'une position réelle (XRL) du chariot distributeur (2) sur la flèche (10) ;
et dans lequel, pour chaque opération de remise en tension du câble métallique de distribution (32), la détermination d'une valeur d'allongement (ΔL) du câble métallique de distribution (32) consiste à établir la valeur d'allongement (ΔL) du câble métallique de distribution (32) comme correspondant à la différence entre la position théorique (XTH) du chariot distributeur (2) et la position réelle (XRL) du chariot distributeur (2).

10. Procédé d'assistance de maintenance selon la revendication 9, dans lequel, après chaque opération de remise en tension du câble métallique de distribution (32), est mis en œuvre une mise à zéro de la position théorique (XTH) du chariot distributeur (2) afin de calibrer cette position théorique (XTH) du chariot distributeur (2) sur la position réelle (XRL) du chariot distributeur (2).

11. Appareil de levage ou de transport (1) comprenant un câble métallique (32) qui forme une boucle fermée et qui coopère avec un système de remise en tension (4), ledit appareil de levage ou de transport (1) étant **caractérisé en ce qu'**il comprend :
- un système de surveillance (81, 82) pour surveiller des opérations de remise en tension du câble métallique (32) par le système de remise en tension (4), qui interviennent dans la durée de vie du câble métallique (32) ;
- un système de contrôle/commande (6) configuré pour :
- déterminer une valeur d'allongement (ΔL) du câble métallique (32) après chaque opération de remise en tension du câble métallique (32) ;
- estimer une valeur d'allongement global (ΔLG) du câble métallique (32), qui correspond à la somme des valeurs d'allongement (ΔL) du câble métallique (32) déterminées après chaque opération de remise en tension ;
- déterminer un indice d'endommagement (IND) du câble métallique (32) en fonction de la valeur d'allongement global (ΔLG).

12. Appareil de levage ou de transport (1) selon la revendication 11, comprenant un treuil (3) muni d'un tambour d'enroulement (31) et le câble métallique (32) présente deux extrémités fixées sur ce tambour d'enroulement (31), pour assurer un déplacement d'un élément fonctionnel (2) de l'appareil de levage ou de transport (1).

13. Appareil de levage ou de transport (1) selon la revendication 12, dans lequel le câble métallique (32) est composé de deux brins (33, 34) qui s'enroulent sur le tambour d'enroulement (31) et qui sont fixés sur le même élément fonctionnel (2).

14. Appareil de levage ou de transport (1) selon la revendication 12 ou 13, dans lequel le câble métallique est un câble métallique de distribution (32) assurant un déplacement d'un chariot distributeur (2) déplaçable le long d'une flèche (10) de l'appareil de levage ou de transport (1).

15. Appareil de levage ou de transport (1) selon l'une quelconque des revendications 11 à 14, dans lequel le système de contrôle/commande (6) est configuré pour déterminer l'indice d'endommagement (IND) du câble métallique (32) aussi en fonction du nombre d'opérations de remise en tension du câble métallique (32).

16. Appareil de levage ou de transport (1) selon l'une quelconque des revendications 11 à 15, dans lequel le système de contrôle/commande (6) est configuré pour déterminer l'indice d'endommagement (IND) du câble métallique (32) aussi en fonction des intervalles de temps entre les opérations successives de remise en tension du câble métallique (32).

17. Appareil de levage ou de transport (1) selon l'une quelconque des revendications 11 à 16, comprenant un système d'alerte (9) relié au système de contrôle/commande (6), ledit système de contrôle/commande (6) étant configuré pour, après chaque opération de remise en tension du câble métallique (32), comparer l'indice d'endommagement (IND) du câble métallique (32) avec un seuil d'endommagement (LIM) prédéfini, et pour activer le système d'alerte (9) afin d'émettre une alerte si l'indice d'endommagement (IND) du câble métallique (32) dépasse ledit seuil d'endommagement (LIM).

## Patentansprüche

1. Wartungsunterstützungsverfahren zur Unterstützung der Wartung eines Drahtseils (32) eines Hebe- oder Transportgerätes (1), wobei das Wartungsunterstützungsverfahren **dadurch gekennzeichnet ist, dass** das Drahtseil (32) ein Drahtseil ist, das eine geschlossene Schleife bildet und mit einem Spannsystem (4) zusammenwirkt, und dadurch, dass es die folgenden Schritte durchführt:
- Überwachung der Spannvorgänge des Drahtseils (32) durch das Spannsystem (4), die innerhalb der Lebensdauer des Drahtseils (32) vorgenommen werden;
- für jeden Spannvorgang des Drahtseils (32), Bestimmung eines Dehnungswertes (ΔL) des Drahtseils (32);
- Schätzung eines Gesamtdehnungswerts (ΔLG) des Drahtseils (32), welcher der Summe der Dehnungswerte (ΔL) des Drahtseils (32) entspricht, die nach jedem Spannen bestimmt werden;
- Bestimmung eines Beschädigungsindexes (IND) des Drahtseils (32) in Abhängigkeit vom Gesamtdehnungswert (ΔLG).

2. Wartungsunterstützungsverfahren nach Anspruch 1, wobei die Bestimmung des Beschädigungsindex (IND) des Drahtseils (32) auch von der Anzahl an Spannvorgängen des Drahtseils (32) abhängig ist.

3. Wartungsunterstützungsverfahren nach Anspruch 1 oder 2, wobei die Bestimmung des Beschädigungsindexes (IND) des Drahtseils (32) auch von den Zeitintervallen zwischen den aufeinanderfolgenden Spannvorgängen des Drahtseils (32) abhängig ist.

4. Wartungsunterstützungsverfahren nach einem der Ansprüche 1 bis 3, umfassend, nach jedem Spannvorgang des Drahtseils (32), einen Vergleich des Beschädigungsindexes (IND) des Drahtseils (32) mit einer vordefinierten Beschädigungsschwelle (LIM) und eine Ausgabe einer Warnung, wenn der Beschädigungsindex (IND) des Drahtseils (32) die Beschädigungsschwelle (LIM) überschreitet.

5. Wartungsunterstützungsverfahren nach Anspruch 4, wobei der Beschädigungsindex (IND) dem Gesamtdehnungswert (ΔLG) entspricht, und die Beschädigungsschwelle (LIM) einer maximalen Dehnungsschwelle entspricht.

6. Wartungsunterstützungsverfahren nach Anspruch 4 oder 5, wobei die Warnung in Form eines optischen oder akustischen Warnsignals an einer Bedienoberfläche vorliegt.

7. Wartungsunterstützungsverfahren nach einem der Ansprüche 1 bis 6, wobei das Drahtseil (32) zwei an einer gleichen Wickeltrommel (31) einer Winde (3) befestigte Enden aufweist, um für eine Verschiebung eines Funktionselements (2) des Hebe- oder Transportgerätes (1) zu sorgen.

8. Wartungsunterstützungsverfahren nach einem der Ansprüche 1 bis 7, wobei das Drahtseil ein Verteilerdrahtseil (32) ist, das für eine Verschiebung eines entlang eines Auslegers (10) des Hebe- oder Transportgerätes (1) verschiebbaren Verteilerschlittens (2) sorgt.

9. Wartungsunterstützungsverfahren nach Anspruch 8, wobei die Überwachung der Spannvorgänge des Verteilungsdrahtseils (32) umsetzt:
- eine Überwachung einer Verteilwinde (3), die eine Wickeltrommel (31) umfasst, auf die sich ein hinterer Strang (34) und ein vorderer Strang (33) des Verteildrahtseils (32) wickeln, um den Verteilerschlitten (2) zu verschieben, wobei die Überwachung der Verteilwinde (3) eine Bestimmung einer theoretischen Position (XTH) des Verteilerschlittens (2) in Abhängigkeit von einem Wickelmaß des hinteren Strangs (34) oder des vorderen Strangs (33) auf der Wickeltrommel umfasst; und
- eine Überwachung einer Ist-Position (XRL) des Verteilerschlittens (2) auf dem Ausleger (10);
und wobei die Bestimmung eines Dehnungswerts (ΔL) des Verteildrahtseils (32) bei jedem Spannvorgang des Verteildrahtseils (32) darin besteht, einen Dehnungswert (ΔL) des Verteildrahtseils (32) als die Differenz zwischen der theoretischen Position (XTH) des Verteilschlittens (2) und der tatsächlichen Position (XRL) des Verteilerschlittens (2) festzulegen.

10. Wartungsunterstützungsverfahren nach Anspruch 9, wobei nach jedem Spannvorgang des Verteildrahtseils (32) eine Nullstellung der theoretischen Position (XTH) des Verteilerschlittens (2) durchgeführt wird, um diese theoretische Position (XTH) des Verteilerschlittens (2) auf die tatsächliche Position (XRL) des Verteilerschlittens (2) zu kalibrieren.

11. Hebe- oder Transportgerät (1), das ein Drahtseil (32) umfasst, das eine geschlossene Schleife bildet und mit einem Spannsystem (4) zusammenwirkt, wobei das Hebe- oder Transportgerät (1) **dadurch gekennzeichnet ist, dass** es umfasst:
- ein Überwachungssystem (81, 82), um Spannvorgänge des Drahtseils (32) durch das Spannsystem (4) zu überwachen, die während der Lebensdauer des Drahtseils (32) vorgenommen werden;
- ein Kontroll-/Steuerungssystem (6), das konfiguriert ist, um:
- einen Dehnungswert (ΔL) des Drahtseils (32) nach jedem Spannvorgang des Drahtseils (32) zu bestimmen;
- einen Gesamtdehnungswert (ΔLG) des Drahtseils (32) zu schätzen, welcher der Summe der Dehnungswerte (ΔL) des Drahtseils (32) entspricht, die nach jedem Spannvorgang bestimmt werden;
- einen Beschädigungsindex (IND) des Drahtseils (32) in Abhängigkeit vom Gesamtdehnungswert (ΔLG) zu bestimmen.

12. Hebe- oder Transportgerät (1) nach Anspruch 11, umfassend eine Winde (3), die mit einer Wickeltrommel (31) versehen ist, und das Drahtseil (32) zwei an dieser Wickeltrommel (31) befestigte Enden aufweist, um für eine Verschiebung eines Funktionselements (2) des Hebe- oder Transportgeräts (1) zu sorgen.

13. Hebe- oder Transportgerät (1) nach Anspruch 12, wobei sich das Drahtseil (32) aus zwei Strängen (33, 34) zusammensetzt, die sich auf die Wickeltrommel (31) aufwickeln und an dem gleichen Funktionselement (2) befestigt sind.

14. Hebe- oder Transportgerät (1) nach Anspruch 12 oder 13, wobei das Drahtseil ein Verteildrahtseil (32) ist, das für eine Verschiebung eines Verteilwagens (2) sorgt, der entlang eines Auslegers (10) des Hebe- oder Transportgeräts (1) verschiebbar ist.

15. Hebe- oder Transportgerät (1) nach einem der Ansprüche 11 bis 14, wobei das Kontroll-/Steuerungssystem (6) konfiguriert ist, um den Beschädigungsindex (IND) des Drahtseils (32) auch in Abhängigkeit von der Anzahl an Spannvorgängen des Drahtseils (32) zu bestimmen.

16. Hebe- oder Transportgerät (1) nach einem der Ansprüche 11 bis 15, wobei das Kontroll-/Steuerungssystem (6) konfiguriert ist, um den Beschädigungsindex (IND) des Drahtseils (32) auch in Abhängigkeit von den Zeitintervallen zwischen den aufeinanderfolgenden Spannvorgängen des Drahtseils (32) zu bestimmen.

17. Hebe- oder Transportgerät (1) nach einem der Ansprüche 11 bis 16, umfassend ein Warnsystem (9), das mit dem Kontroll-/Steuerungssystem (6) verbunden ist, wobei das Kontroll-/Steuerungssystem (6) konfiguriert ist, um nach jedem Spannvorgang des Drahtseils (32) den Beschädigungsindex (IND) des Drahtseils (32) mit einer vordefinierten Beschädigungsschwelle (LIM) zu vergleichen und um das Warnsystem (9) zu aktivieren, um eine Warnung auszugeben, wenn der Beschädigungsindex (IND) des Drahtseils (32) die Beschädigungsschwelle (LIM) überschreitet.

## Claims

1. A maintenance assistance method for assisting in maintaining a metal cable (32) of a lifting or transport apparatus (1), said maintenance assistance method being **characterized in that** said metal cable (32) is a metal cable forming a closed loop and cooperating with a re-tensioning system (4), and **in that** it implements the following steps:
- supervision of the re-tensioning operations of the metal cable (32) by the re-tensioning system (4), which occur in the lifetime of the metal cable (32);
- for each operation of re-tensioning the metal cable (32), determination of an elongation value (ΔL) of the metal cable (32);
- estimation of an overall elongation value (ΔLG) of the metal cable (32), which corresponds to the sum of the elongation values (ΔL) of the metal cable (32) determined after each re-tensioning operation;
- determination of a damage index (IND) of the metal cable (32) as a function of the overall elongation value (ΔLG).

2. The maintenance assistance method according to claim 1, wherein the determination of the damage index (IND) of the metal cable (32) depends also on the number of re-tensioning operations of the metal cable (32).

3. The maintenance assistance method according to claim 1 or 2, wherein the determination of the damage index (IND) of the metal cable (32) is also a function of the time intervals between the successive re-tensioning operations of the metal cable (32).

4. The maintenance assistance method according to any one of the claims 1 to 3, comprising, after each re-tensioning operation of the metal cable (32), a comparison of the damage index (IND) of the metal cable (32) with a predefined damage threshold (LIM), and an issuing of an alert if the damage index (IND) of the metal cable (32) exceeds said damage threshold (LIM).

5. The maintenance assistance method according to claim 4, wherein the damage index (IND) corresponds to the overall elongation value (ΔLG), and the damage threshold (LIM) corresponds to an elongation maximum threshold.

6. The maintenance assistance method according to claim 4 or 5, wherein the alert is in the form of a visual or audible alert signal on a control interface.

7. The maintenance assistance method according to any one of the claims 1 to 6, wherein the metal cable (32) has two ends fastened to the same winding drum (31) of a winch (3), for ensuring a displacement of a functional element (2) of the lifting or transport apparatus (1).

8. The maintenance assistance method according to any one of the claims 1 to 7, wherein the metal cable is a distribution metal cable (32) ensuring a displacement of a distribution trolley (2) displaceable along a jib (10) of the lifting or transport apparatus (1).

9. The maintenance assistance method according to claim 8, wherein the supervision of the re-tensioning operations of the distribution metal cable (32) implements:
- a supervision of a distribution winch (3) comprising a winding drum (31) on which are wound a rear strand (34) and a front strand (33) of the distribution metal cable (32) to displace the distribution trolley (2), said supervision of the distribution winch (3) comprising a determination of a theoretical position (XTH) of the distribution trolley (2) as a function of a measure of winding of the rear strand (34) or of the front strand (33) on the winding drum; and
- a supervision of an actual position (XRL) of the distribution trolley (2) on the jib (10);
and wherein, for each re-tensioning operation of the distribution metal cable (32), the determination of an elongation value (ΔL) of the distribution metal cable (32) consists in establishing the elongation value (ΔL) of the distribution metal cable (32) as corresponding to the difference between the theoretical position (XTH) of the distribution trolley (2) and the actual position (XRL) of the distribution trolley (2).

10. The maintenance assistance method according to claim 9, wherein, after each re-tensioning operation of the distribution metal cable (32), a resetting of the theoretical position (XTH) of the distribution trolley (2) is implemented in order to calibrate this theoretical position (XTH) of the distribution trolley (2) on the actual position (XRL) of the distribution trolley (2).

11. A lifting or transport apparatus (1) comprising a metal cable (32) which forms a closed loop and which cooperates with a re-tensioning system (4), said lifting or transport apparatus (1) being **characterized in that** it comprises:
- a supervision system (81, 82) for supervising re-tensioning operations of the metal cable (32) by the re-tensioning system (4), which occur in the lifetime of the metal cable (32);
- a monitoring/control system (6) configured to:
- determine an elongation value (ΔL) of the metal cable (32) after each re-tensioning operation of the metal cable (32);
- estimate an overall elongation value (ΔLG) of the metal cable (32), which corresponds to the sum of the elongation values (ΔL) of the metal cable (32) determined after each re-tensioning operation;
- determine a damage index (IND) of the metal cable (32) as a function of the overall elongation value (ΔLG).

12. The lifting or transport apparatus (1) according to claim 11, comprising a winch (3) provided with a winding drum (31) and the metal cable (32) has two ends fastened to this winding drum (31), to ensure a displacement of a functional element (2) of the lifting or transport apparatus (1).

13. The lifting or transport apparatus (1) according to claim 12, wherein the metal cable (32) is composed of two strands (33, 34) which are wound on the winding drum (31) and which are fastened on the same functional element (2).

14. The lifting or transport apparatus (1) according to claim 12 or 13, wherein the metal cable is a distribution metal cable (32) ensuring a displacement of a distribution trolley (2) displaceable along a jib (10) of the lifting or transport apparatus (1).

15. The lifting or transport apparatus (1) according to any one of the claims 11 to 14, wherein the monitoring/control system (6) is configured to determine the damage index (IND) of the metal cable (32) as a function of the number of re-tensioning operations of the metal cable (32).

16. The lifting or transport apparatus (1) according to any one of the claims 11 to 15, wherein the monitoring/control system (6) is configured to determine the damage index (IND) of the metal cable (32) also as a function of the time intervals between the successive re-tensioning operations of the metal cable (32).

17. The lifting or transport apparatus (1) according to any one of the claims 11 to 16, comprising an alert system (9) connected to the monitoring/control system (6), said monitoring/control system (6) being configured to, after each re-tensioning operation of the metal cable (32), compare the damage index (IND) of the metal cable (32) with a predefined damage threshold (LIM), and to activate the alert system (9) in order to issue an alert if the damage index (IND) of the metal cable (32) exceeds said damage threshold (LIM).
